# EUROPEAN PATENT APPLICATION

(11) **EP 1 040 835 A2**
(43) Date of publication of application: **04.10.2000**
(21) Application number: 00301682.1
(22) Date of filing: 01.03.2000
(51) Int. Cl.: A61K 38/18, A61K 48/00

(54) **Use of HST-1 in preventing and/or treating tissue damage in cancer therapy**

(30) Priority: 01.03.1999 JP 5338999
(71) Applicant: Sumitomo Pharmaceuticals Company, Limited, Osaka-shi, Osaka 541-8510 (JP)
(72) Inventor: Ochiya, Takahiro, Nat.Cancer C. R. Instit. Tsukiji, Chuo-ku, Tokyo 104-0045 (JP); Terada, Masaki, Nerima-ku, Tokyo 177-0053 (JP); Sakamoto, Hiromi, Hanamigawa-ku, Chiba 262-0022 (JP); Takahama, Yasushi, Nosekogawa-mura Nat.Ins.Cl.Dr., Yoshino-gun, Nara 637-0424 (JP)
(74) Representative: Keen, Celia Mary

(57) **Abstract**

An HST-1 DNA construct, HST-1 protein and an equivalent thereof enhance proliferation and/or differentiation of stem cells, germ cells, hair-bulb cells, intestinal cells, villus, mucosa, glomerulus endothelial cells or spleen cells, and therefore are useful as a medicament for preventing and/or treating tissue damage caused by an anti-cancer therapy or irradiation.

## Description

### FIELD OF THE INVENTION

The present invention relates to a medicament for preventing and/or treating organ damage caused by an anti-cancer therapy or irradiation comprising heparin-binding secretory transforming factor (which is identical to FGF-4 and referred to as HST-1 hereafter) or an HST-1 gene, etc.

### BACKGROUND OF THE INVENTION

The HST-1 gene was originally identified by the present inventors as a transforming gene from human gastric tumor, and is located on human chromosome 11q13 (Proc. Natl. Acad. Sci. USA, 83, 3997-4001 (1986); *ibid*. , 84, 2980-2984 (1987) ; Methods Enzymol., 198, 124-138 (1991)). Unlike acidic and basic FGFs, HST-1 protein is easily secreted out of the cells since the HST-1 gene contains a signal peptide (Proc. Natl. Acad. Sci. USA, 84, 2980-2984 (1987)). Therefore, the HST-1 protein may act on target cells effectively.

HST-1 protein acts on the megakaryoblast in bone marrow and differentiates them to induce them to produce platelets. US 5,559,093 discloses that the HST-1 protein and their deletion mutein increase platelet production to restore the number of platelets decreased by thrombocytopenia, anticancer drugs or irradiation. J. Clin. Invest., 96, 1125-1130 (1995) and Leukemia, 11, 530-532 (1997) disclose that an adenovirus vector containing a human HST-1 gene may be applied for thrombocytopenia as a gene therapy.

It is not known that the HST-1 protein and HST-1 gene enhance proliferation and/or differentiation of stem cells, germ cells, hair-bulb cells, intestinal cells, villus, mucosa, glomerulus endothelial cells and spleen cells where the stem cells are existing.

### SUMMARY OF THE INVENTION

The present invention is intended to provide a medicament for preventing and/or treating tissue damage caused by an anti-cancer therapy or irradiation. The present inventors found that the HST-1 gene enhanced production of various tissues or cells which differentiated and/or proliferate actively, especially it may rely on stem cells. Thus, the present invention has been accomplished.

That is, the present invention is as follows.
[1] A medicament for enhancing proliferation and/or differentiation of stem cells, germ cells, hair-bulb cells, intestinal cells, villus, mucosa, glomerulus endothelial cells or spleen cells, comprising an HST-1 DNA construct, HST-1 protein or an equivalent thereof.
[2] A medicament for preventing and/or treating tissue damage caused by an anti-cancer therapy or irradiation, comprising an HST-1 DNA construct, HST-1 protein or an equivalent thereof, provided that the tissue damage is not thrombocytopenia.
[3] A medicament according to [2] wherein the anti-cancer therapy is radiation therapy.
[4] A medicament according to [2] or [3] wherein the tissue is that composed of hematopoietic stem cells or intestinal stem cells.
[5] A medicament according to [2] or [3] wherein the tissue is bone marrow or intestinal tract.
[6] A medicament according to [2] or [3] wherein the tissue damage is leukopenia, anemia, oligocythemia or mucositis.
[7] A medicament according to [2] or [3] wherein the tissue damage is necrosis of bone marrow or suppression of peripheral blood cells.
[8] A medicament according to [2] or [3] wherein the tissue damage is intestinal hemorrhage, suppression of villus or secondary infection.
[9] A medicament comprising an HST-1 DNA construct according to any one of [1] to [8].
[10] A medicament according to [9] wherein the HST-1 DNA construct is introduced into a vector.
[11] A medicament according to [9] wherein the HST-1 DNA construct is introduced into an adenovirus vector or a plasmid vector.

### BRIEF DESCRIPTION OF THE DRAWINGS

Fig. 1 shows the probability of survival of irradiated mice in the test of preventive effect: Example 2(2).

Fig. 2 shows a histological section (x ca. 40) of bone marrow of the control group in the test of preventive effect: Example 2(3).

Fig. 3 shows a histological section (x ca. 40) of bone marrow of the test group in the test of preventive effect: Example 2(3).

Fig. 4 shows a histological section (x ca. 200) of bone marrow of the control group in the test of preventive effect: Example 2(3).

Fig. 5 shows a histological section (x ca. 200) of bone marrow of the test group in the test of preventive effect: Example 2(3).

Fig. 6 shows a histological section (x ca. 40) of the spleen of the control group in the test of preventive effect: Example 2(3).

Fig. 7 shows a histological section (x ca. 40) of the spleen of the test group in the test of preventive effect: Example 2(3).

Fig. 8 shows a histological section (x ca. 40) of the intestines of the control group in the test of preventive effect: Example 2(3).

Fig. 9 shows a histological section (x ca. 40) of the intestines of the test group in the test of preventive effect: Example 2(3).

Fig. 10 shows the probability of survival of irradiated mice in the test of therapeutic effect: Example 3(2).

### DETAILED DESCRIPTION OF THE INVENTION

"HST-1 DNA construct" is a construct comprising an HST-1 DNA or an equivalent thereof and additional sequence(s) necessary for expressing HST-1 protein or an equivalent thereof.

"HST-1 DNA" is a DNA comprising the human HST-1 cDNA sequence described in Proc. Natl. Acad. Sci. USA., 84, 2980-2984 (1987).

"Equivalent of HST-1 DNA" includes, for example, DNA encoding a protein which may contain insertion, deletion and/or substitution of one or more amino acids in the "HST-1 protein" and which has a similar activity to that of "HST-1 protein", and DNA which hybridizes under a stringent condition to "HST-1 DNA" and which encodes a protein having a similar activity to that of "HST-1 protein".

"Additional sequence" includes for example promoters and bacterial origin of replication sequences such as the SV40 early poly A signal etc. Promoters may be selected with the knowledge of level of expression and tissue specificity. Examples of promoters include SRα promoter (Mol. Cell. Biol., 8, 466-472 (1988)), cytomegalovirus promoter (CMV) and Rous sarcoma virus long terminal repeat promoter (RSV-LTR). If a high level of expression is not necessary, mouse mammary tumor virus promoter (MMTV-LTR), mouse metallotionein-1 promoter (mMT-1) etc. may be useful.

"HST-1 protein" is the HST-1 protein described in Proc. Natl. Acad. Sci. USA. , 84, 2980-2984 (1987). "Equivalent of HST-1 protein" includes, for example, a protein which may be produced by expression of "equivalent of HST-1 DNA" and is different from "HST-1 protein". "HST-1 protein" and "Equivalent of HST-1 protein" may have at least one sugar chain, lipid or acetyl group, as long as it has a similar activity to that of "HST-1 protein". Examples of "equivalent, of HST-1 protein" are proteins which have amino acid sequence in which 27, 30, 31, 43, 46 and 47 amino acids are eliminated from the N-terminus of the 206 amino acid sequence of "HST-1 protein" (Mol. Cell. Biol. , 8, 2933-2941 (1988) ; US 5, 639, 862).

The activity of "HST-1 protein" may be measured, for example, by stimulation of DNA synthesis of mouse BALB/c3T3 cells based upon the uptake of [³H]-thymidine (Mol. Cell. Biol. 8, 588-594 (1988)), growth promotion of the vascular endothelial cells (J. Immun. Methods, 93, 157 (1986)), or angiogenesis on the avian embryo allantois (Dev. Biol. , 41, 391 (1974)).

"Anti-cancer therapy" includes chemotherapy and radiation therapy. Examples of chemotherapeutic agents are alkylating agents such as nitrogen mustard N-oxide, cyclophosphamide, melphalan, carboquone, busulfan, nimustine, ranimustine and dacarbazine; antimetabolites such as fluorouracil, tegaful, cytarabine, ancitabine, broxuridine, doxifluridine, mercaptopurine, thioinosine, azathioprine and methotrexate; antibiotics such as mitomycin, bleomycin, daunorubicin, doxorubicin, pirarubicin, aclarubicin, neocarzinostatin and actinomycin D; alkaloids such as vincristine, vindesine, vinblastine and etoposide; hormone such as tamoxifen; platinum such as cisplatin and carboplatin; procarbazine, mitobronitol, mitoxanton, chlorambucil etc.

Anti cancer therapies usually cause damage to tissues and cells which actively proliferate and/or differentiate. Examples of such tissues and cells are various stem cells (e. g. hematopoietic stem cells, myeloid stem cells, lymphoid stem cells, intestinal stem cells, neural stem cells, etc.), germ cells, hair-bulb cells, intestinal cells, villus, mucosa, glomerulus endothelial cells or spleen cells. Damage to those tissues and/or cells includes necrosis, atrophy and/or degeneration of the tissues and organs including the tissues or the cells, for example, necrosis of bone marrow, hemorrhage from the intestine, degeneration of villus, immune suppression followed by secondary infection etc. And finally these damage might cause death.

The medicament of the present invention can enhance proliferation and/or differentiation of stem cells, germ cells, hair-bulb cells, intestinal cells, villus, mucosa, glomerulus endothelial cells and spleen cells, and thus is useful as a medicament for preventing and/or treating tissue damage caused by an anti-cancer therapy and irradiation. Since this medicament has a preventive effect for such damage, anti-cancer therapy can be executed more efficiently. This medicament is useful as a medicament for mucositis (e.g. intestinal mucositis, oral mucositis), leukopenia (e.g. neutropenia, eosinopenia, lymphopenia), anemia and oligocythemia. This medicament is also useful for supporting bone marrow transplantation therapy.

"HST-1 DNA construct" can be applied as gene therapy. The gene therapy is usually classified into two types: *in vivo method* and *ex vivo* method (NIKKEI Science, 1994(4), 20-45; GEKKAN-YAKUJI, 36(1), 23-48 (1994); JIKKEN-IGAKU, 12(extra issue 15), 1298-1933 (1994) and references cited therein). "HST-1 DNA construct" can be employed by administering "HST-1 DNA construct" directly to a body (*In vivo* method) and by collecting certain cells from a body, introducing "HST-1 DNA construct" into the cells and finally returning the cells to the body *(Ex viv*o method).

For the *in vivo* method, there may be used the viral vector method, liposome vector method, fusogenic liposome vector method, lipofectin vector method etc. (NIKKEI Science, 1994(4), 20-45; GEKKAN-YAKUJI, 36(1), 23-48 (1994); JIKKEN-IGAKU, 12(extra issue 15), 1298-1933 (1994); Cardiovascular Research, 28, 445 (1994); Science, 256, 808 (1992); Gastroenterology, 106, 1076 (1994); TIBTECH, 11, 182, (1993); J. Biol. Chem., 266, 311 (1991); Nature Medicine, 1, 583 (1995); and references cited therein).

The "viral vector method" may be executed by introducing "HST-1 DNA construct" into a DNA viral vector (e.g. an adenovirus vector, an adeno-associated virus (AAV) vector, a Herpes virus vector, a vaccinia virus vector, a pox virus vector, a polio virus vector, a sindbis virus vector, a retrovirus vector, Sendai virus (HVJ: hemagglutinating virus of Japan) vector etc.) or a RNA viral vector. Preferable examples of the viral vectors are adenovirus vectors such as adenovirus type-5 (ATCC No. VR-5), because adenovirus has high titers of virus, is stable and easy to handle, and infects non-dividing cells.

Liposome vectors used in "liposome vector method" are usually made from phospholipids. Examples of the phospholipids are phosphatidylcholines (e.g. lecithin, lysolecithin, etc.), acidic phopholipids (e.g. phosphatidylserine, phosphatidylic acid, etc.), phospholipids obtained by replacing an acyl group(s) of these phospholipids with lauroyl, myristoyl, oleoyl, etc. and sphingophosphilipids such as phosphatidylethanolamine, sphingomyelin, etc. Neutral lipids such as cholesterol may also be added to these phospholipids. The liposome vectors may be prepared in a conventional manner, from naturally occurring materials such as lipids in normal cell membranes. The liposome vector containing an HST-1 DNA construct may be prepared for example by suspending a thin layer of purified phospholipids in an aqueous solution of the HST-1 DNA construct and then treating the suspension in a conventional manner such as ultrasonication. The HST-1 DNA construct may be in any form that can express the DNA in a body, but is preferably a form stable in a body such as a plasmid, etc.

Fusogenic liposome vectors used in "fusogenic liposome vector method" include liposome vectors fused with viruses, typically a liposome vector fused with HVJ (NIKKEI Science, 1994(4), 32-38; J. Biol. Chem., 266(6), 3361-3364 (1991)). The liposome vector fused with HVJ may be prepared by mixing purified HVJ inactivated by ultraviolet irradiation, etc. with a liposome suspension containing an HST-1 DNA construct, gently agitating the mixture and then removing unbound HVJ by sucrose density gradient centrifugation. The liposome vectors may be bound to substances having an affinity for gene introduction into a target cells. The HST-1 DNA construct may be in any form that can express the DNA in a body, but is preferably in a form which is stable in a body such as a plasmid, etc.

In the *in vivo* method, "HST-1 DNA construct" can usually be administered intravenously, and may be also administered intraarterially, subcutaneously, intradermally, intramuscularly, by local injection, intraperitoneally, orally, intrarectaliy, percutaneously or intranasally. Pharmaceutical formulations for intravenous administration include for example injections such as solutions, suspensions, etc. The formulations can be prepared by the conventional methods. "HST-1 DNA construct" can be dissolved or suspended in a physiologically acceptable carrier or diluent such as distilled water, physiological saline, dextrose solution or the like, which may contain auxiliary agents such as pH adjusters, buffers (sodium acetate, sodium lactate, sodium citrate etc.), stabilizers (human serum albumin, sorbitol, polyethylene glycol, amino acid etc.), solubilizers (sorbitan monolaurate, triethanolamine oleate etc.), isotonic agents (sodium chloride, potassium chloride, calcium chloride, glucose etc. ), preservatives (benzalkonium chloride, benzyl alcohol, phenol etc.), soothing agents (procaine hydrochloride, etc.) and the like, if desired. The solutions or suspensions may be packaged as they are. The solutions may be also lyophilized and be dissolved in sterile saline before administration. The formulations may take the form of sustained release preparations using biocompatible materials such as collagen, and can be prepared by conventional methods (EP 844004, WO99/61063).

The dose and the frequency for administration of HST-1 DNA construct in the *in vivo* method varies widely depending on the species to be cured, the severity of the symptoms, the patient's age, body weight, sex, administration route, and the like. "HST-1 DNA construct" is usually administered to an adult (body weight 60 kg) in a dose of approximately about 0.0001 mg to about 100 mg, preferably about 0.0005 mg to about 50 mg, more preferably about 0.001 mg to about 10 mg per day in one portion or several portions or by continuous drip infusion. They may be also administered once every 2 days to once every 2 months.

For the *ex vivo* method, introduction of "HST-1 DNA construct" into the cells may be executed by micro-injection method, calcium phosphate method, electroporation method etc. Generally the introduction may be preferably done using a retrovirus vector (Human Gene Therapy, 2, 251-256 (1991); Science, 243, 220-222 (1989)).

The *in vivo* method is usually more preferable than the *ex vivo* method, because the *in vivo* method *is* less costly, more convenient and it is easier to control the duration of expression.

The applied HST-1 DNA construct expresses in the body to produce HST-1 protein or an equivalent thereof, which exhibits its therapeutic and/or preventive effect. Usually HST-1 DNA construct works longer than HST-1 protein or an equivalent thereof.

"HST-1 protein" and "equivalent of HST-1 protein" can be usually administered intravenously, and may be also administered intraarterially, subcutaneously, intradermally, intramuscularly, by local injection, intraperitoneally, orally, intrarectally, percutaneously or intranasally. Pharmaceutical formulations for intravenous administration include for example injections such as solutions, suspensions, etc. The formulations can be prepared by conventional methods. "HST-1 protein" or "equivalent of HST-1 protein" can be dissolved or suspended in a physiologically acceptable carrier or diluent such as distilled water, physiological saline, dextrose solution or the like, which may contain auxiliary agents such as pH adjusters, buffers (sodium acetate, sodium lactate, sodium citrate etc.), stabilizers (human serum albumin, sorbitol, polyethylene glycol, amino acid etc.), solubilizers (sorbitan monolaurate, triethanolamine oleate etc.), isotonic agents (sodium chloride, potassium chloride, calcium chloride, glucose etc. ), preservatives (benzalkonium chloride, benzyl alcohol, phenol etc.), soothing agents (procaine hydrochloride, etc.) and the like, if desired. The solutions or suspensions may be packaged as they are. The solutions may be also lyophilized and be dissolved in sterile saline before administration.

Pharmaceutical formulations for subcutaneous, intramuscular, local injection, intraperitoneal, intrarectal, percutaneous and intranasal administration include for example injections such as solutions, suspensions and emulsions, suppositories for administration through the rectum, dermal formulations (ointments, creams, lotions, etc.) and the like. These formulations may take the form of sustained release preparations using biocompatible materials such as collagen, and can be prepared by conventional methods.

Pharmaceutical formulations for oral administration include for example powders, tablets, pills, capsules, granules, fine granules, suspensions, emulsions, syrups and the like. The formulations can be prepared by conventional methods using conventional carriers or diluents.

The dose and the frequency for administration varies widely depending on the species to be cured, the severity of the symptoms, the patient's age, body weight, sex, administration route, and the like. "HST-1 protein" or "equivalent of HST-1 protein" is usually administered to an adult (body weight 60 kg) in a dose of approximately about 0.0001 mg to about 200 mg, preferably about 0.0005 mg to about 100 mg, more preferably about 0. 002 mg to about 50 mg per day in one portion or several portions or by continuous drip infusion. They may be also administered once every 2 days to once every 1 month.

### EXAMPLES

The present invention is precisely explained below with examples and experiments but is, of course, not limited by them.

### Example 1

### Introduction of HST-1 cDNA to an adenovirus vector

E1A, E1B and E3 regions of adenovirus type 5 (ATCC No. VR-5) were deleted. SRα promoter (Mol. Cell. Biol., 8, 466-472 (1988)), human HST-1 cDNA (Proc. Natl. Acad. Sci. USA, 84, 2980-2984 (1987)) and the SV40 early poly A signal were inserted into these regions according to an *in vivo* homologous recombination method (J. Virol., 54, 711-719 (1985); Proc. Natl. Acad. Sci. USA, 93, 1320 (1996); Cell, 13, 181-188 (1978)). 293 cells were infected with the adenovirus vector obtained and incubated until the 293 cells became confluent. Viral supernatants were harvested by 5 freeze-thaw cycles, followed by concentration by ultracentrifugation through two cesium chloride gradients (Jpn. Med. Sci. Biol., 7, 157-166 (1994)). After being desalted, 1 x 10¹⁰ pfu (plaque forming unit)/mL of the adenovirus vector was frozen at -80°C.

### Example 2

### Preventive effect of HST-1 gene on radiated mice

Eight-week-old female C57BL/6 mice were divided into 3 groups (15 mice in each group). The adenovirus vector of Example 1 (10⁹ pfu/mouse) was injected intraperitoneally to mice of the test group 3 days before irradiation. To mice of the control group was injected wild type adenovirus at the same time. Mice of the test and control groups were exposed under 9 Gy (900 rad) irradiation, which is a 100% lethal dose. Hematological parameters were measured 20 days after the irradiation. Probability of survival was observed till 30 days after the irradiation. Histological analyses of bone marrow, the spleen and the intestines and the intestine hemorrhage were observed 14 days after the irradiation.

### (1) Hematological parameters

**Table 1**

| | Unirradiated | Control group | Test group |
|---|---|---|---|
| Hemoglobin (g/dL) | 16.7 | 2.1 | 13.2 |
| Red blood cells (10⁴/mm³) | 1,056 | 30 | 880 |
| White blood cells (1/mm³) | 11,400 | 0 | 1,000 |
| Platelets (10⁴/mm³) | 29.6 | 3 | 28.0 |

As shown in Table 1, the control group lost almost every type of blood cell after irradiation. On the other hand, the test group recovered a number of blood cells, especially white blood cells, when compared with the control group.

### (2) Probability of survival

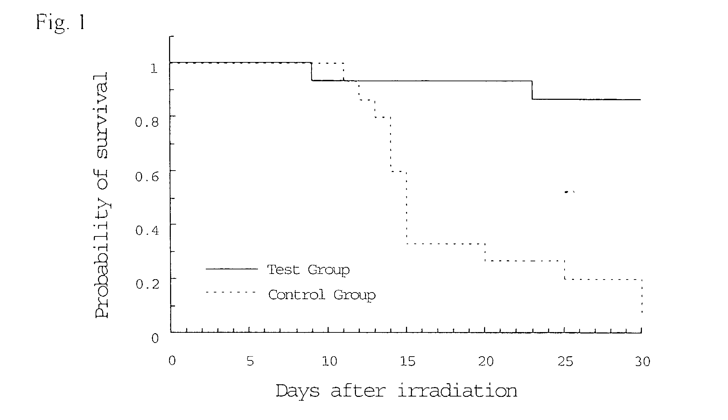

The probability of survival is shown in Fig. 1. Thirteen in 15 mice survived in the test group 30 days after the irradiation, while only one in 15 mice survived in the control group. The HST-1 gene has efficient survival effect.

### (3) Histological analyses of bone marrow, the spleen and the intestines

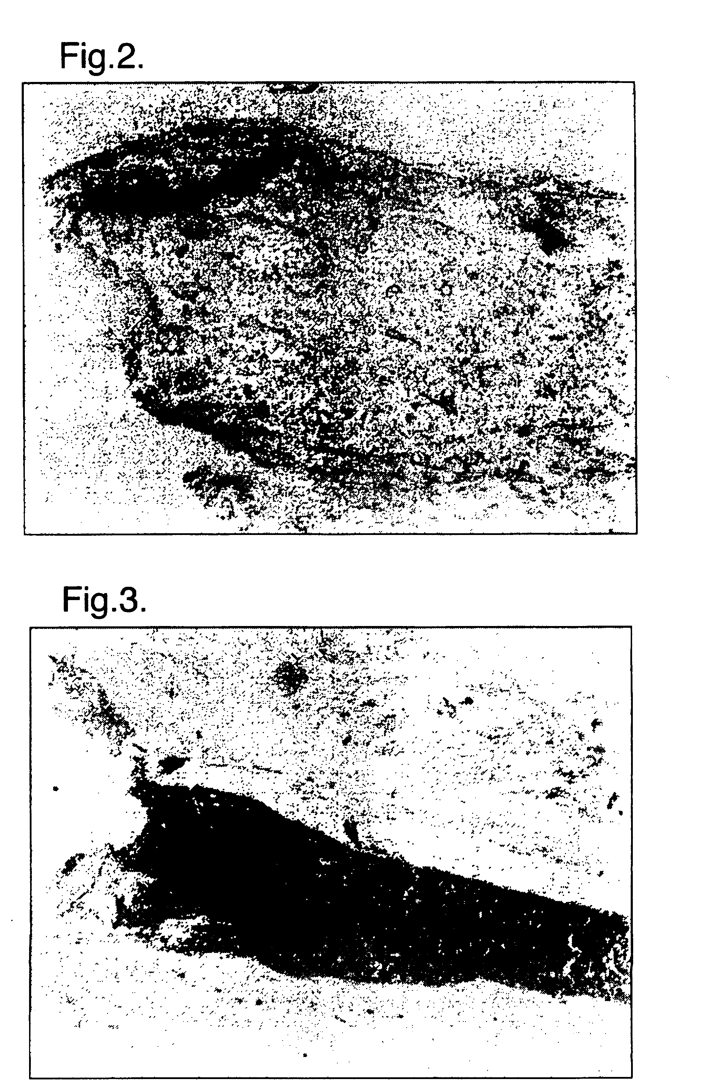

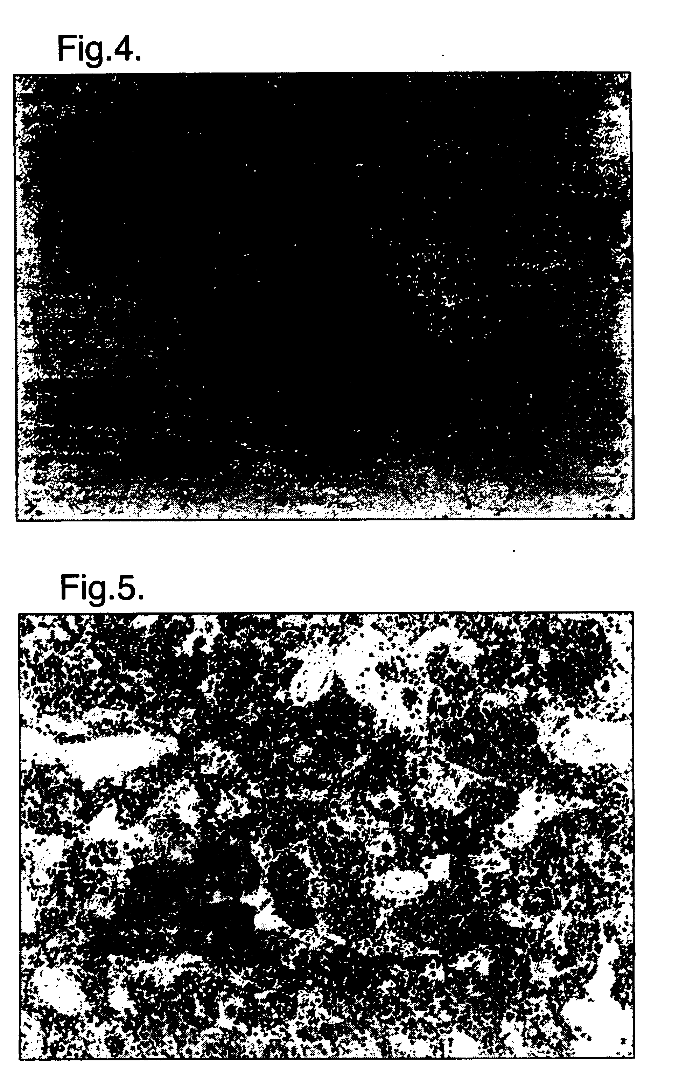

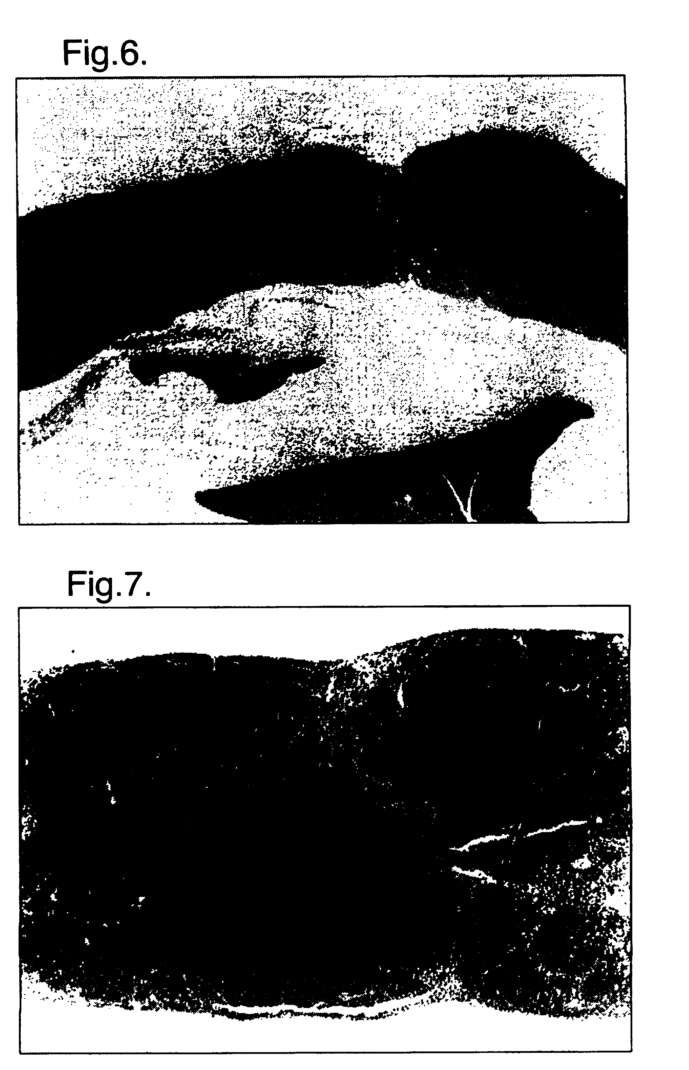

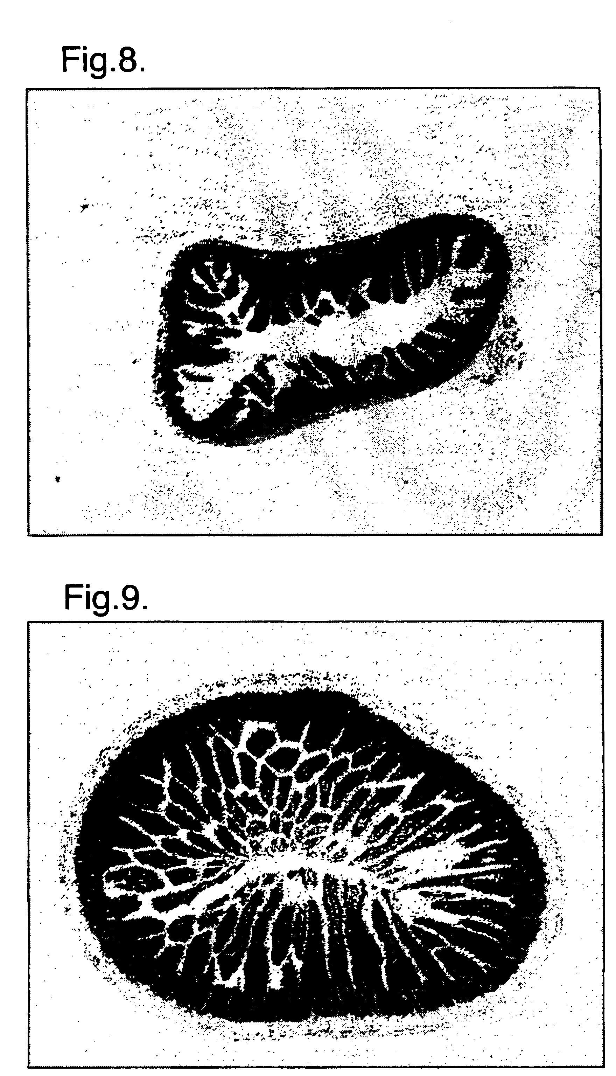

Histological sections of bone marrow, the spleen and the intestines are shown in Fig. 2 to Fig. 9. In the control group, necrosis, atrophy and degeneration were observed widely. Bone marrow was degenerated severely and the spleen was atrophied remarkably. On the other hand, the structure of tissues looked normal, and necrosis, atrophy and degeneration were rarely observed in the test group.

The intestines bled heavily in the control group, while hemorrhage in the test group was not observed.

These data indicate that an HST-1 gene and HST-1 protein, etc., have a preventive effect against tissue damage caused by irradiation.

### Example 3

### Therapeutic effect of HST-1 gene on radiated mice

Eight-week-old female C57BL/6 mice were divided into 3 groups (10 mice in each group). Mice of test and control groups were exposed under 9 Gy (900 rad) irradiation, which is a 100% lethal dose. The adenovirus vector of Example 1 (10⁹ pfu/mouse) was injected intraperitoneally to mice of the test group 6 hours after irradiation. To mice of the control group was injected wild type adenovirus at the same time. Hematological parameters were measured 20 days after the irradiation. Probability of survival was observed till 30 days after the irradiation.

### (1) Hematological parameters

**Table 2**

| | Unirradiated | Control group | Test group |
|---|---|---|---|
| Hemoglobin (g/dL) | 16.1 | 1.2 | 7.2 |
| Red blood cells (10⁴/mm³) | 1,022 | 20 | 580 |
| White blood cells (1/mm³) | 10,900 | 0 | 1,900 |
| Platelets (10⁴/mm³) | 28.4 | 4 | 13.0 |

As shown in Table 2, the control group lost almost every type of blood cell after the irradiation. On the other hand, the test group recovered nearly half the number of blood cells and platelets, especially white blood cells.

### (2) Probability of survival

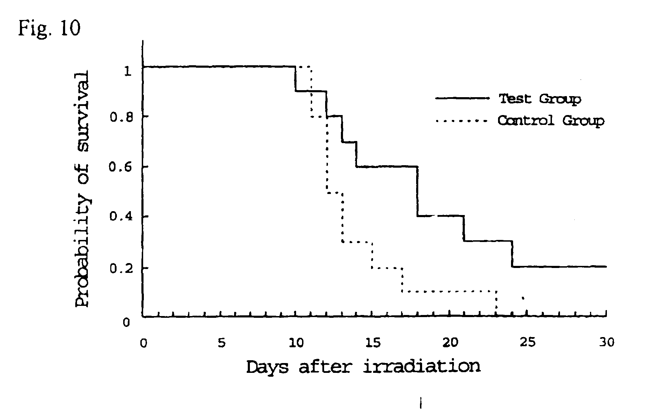

The probability of survival is shown in Fig. 10. Two in 10 mice survived in the test group 30 days after the irradiation, while all mice in the control group died before day 23. The HST-1 gene has an efficient survival effect.

This data indicate that an HST-1 gene and HST-1 protein, etc., have a therapeutic effect against. tissue damage caused by the irradiation.

## Claims

1. Use of an HST-1 DNA construct, HST-1 protein or an equivalent thereof for the manufacture of a medicament for enhancing proliferation and/or differentiation of stem cells, germ cells, hair-bulb cells, intestinal cells, villus, mucosa, glomerulus endothelial cells or spleen cells.

2. Use of an HST-1 DNA construct, HST-1 protein or an equivalent thereof for the manufacture of a medicament for preventing and/or treating tissue damage caused by an anti-cancer therapy or irradiation, provided that the tissue damage is not thrombocytopenia.

3. Use according to Claim 2 wherein the anti-cancer therapy is radiation therapy.

4. Use according to Claim 2 or Claim 3 wherein the tissue is that composed of hematopoietic stem cells or intestinal stem cells.

5. Use according to Claim 2 or Claim 3 wherein the tissue is bone marrow or intestinal tract.

6. Use according to Claim 2 or Claim 3 wherein the tissue damage is leukopenia, anemia, oligocythemia or mucositis.

7. Use according to Claim 2 or Claim 3 wherein the tissue damage is necrosis of bone marrow or suppression of peripheral blood cells.

8. Use according to Claim 2 or Claim 3 wherein the tissue damage is intestinal hemorrhage, suppression of villus or secondary infection.

9. Use of an HST-1 DNA construct according to any one of Claim 1 to Claim 8.

10. Use according to Claim 9 wherein the HST-1 DNA construct is introduced into a vector.

11. Use according to Claim 9 wherein the HST-1 DNA construct is introduced into an adenovirus vector or a plasmid vector.
